# EUROPEAN PATENT APPLICATION

(11) **EP 3 812 004 A1**
(43) Date of publication of application: **28.04.2021**
(21) Application number: 19208583.5
(22) Date of filing: 12.11.2019
(51) Int. Cl.: A61N 1/375

(54) **HERMETIC FEEDTHROUGH WITH SPIRAL ROLLED CONDUCTOR ASSEMBLY**

(30) Priority: 23.10.2019 US 201962924714 P
(71) Applicant: BIOTRONIK SE & Co. KG, 12359 Berlin (DE)
(72) Inventor: Primavera, Anthony A., Newberg, OR 97132 (US); Whitaker, Colby, Oregon City, OR 97045 (US)
(74) Representative: Galander, Marcus

(57) **Abstract**

The present invention relates to a method for producing an electrical feedthrough (1) for a medical device, comprising the steps of: providing a plurality of conductors (2); winding a layer (3) comprising an electrically insulating material, such that the layer (3) encloses said plurality of conductors (2) to form a subassembly (10) comprising said layer (3) and said plurality of conductors (2); compressing of the subassembly (10), and separating the subassembly (10) into a plurality of disc-shaped electrical feedthroughs (1).

## Description

The present invention relates to a method for producing an electrical feedthrough, to an electrical feedthrough as well as to an implantable medical device comprising such an electrical feedthrough.

All implantable medical devices that provide electrical stimulation or some sort of sensing functionality must have some form of electronics that monitor the patient, provide therapy, or both. The device electronics are nearly universally not biocompatible and will cause adverse reactions with the patient if they were to come in direct or indirect contact with the patient. Typically, a titanium housing is used to separate the device electronics from the patient. The electronics must, however, have some direct electrical connection with the patient. The connection can be made using a hermetically sealed electrical feedthrough that allows for therapy to be applied to the patient from the device electronics and biological signals from the patient to be sensed by the device electronics. The electrical feedthrough component is complex and typically requires manual processes that significantly increase the cost of the feedthrough and can impact the quality of the feedthrough.

Known electrical feedthroughs often require individual interconnects to be brazed to a ceramic structure and typically involve a highly manual process and many components.

Furthermore complex machined shapes are frequently required for supporting components (e.g. titanium flange). Also, electrical feedthroughs regularly comprise form factors that increase the circumferential path length of the electrical feedthrough (e.g. oblong/rectangular in shape) and reduce interconnect density.

Furthermore, solutions using multi-layered ceramic technology require successive layer build up to ensure hermetic properties.

Based on the above, it is an objective of the present invention to design an electrical feedthrough for an implantable medical device that can be manufactured in a simple, preferably semi-continuous process, and enables high density interconnects. Furthermore, it is particularly desirable to achieve low cost manufacture by allowing the use of high volume low cost materials and of a simplified manufacturing procedure.

To this end, a method for producing an electrical feedthrough for an implantable medical device is disclosed, comprising the steps of:
- providing a plurality of conductors,
- winding a layer of an electrically insulating material, such that the layer encloses said plurality of conductors laterally to form a subassembly comprising said layer and said plurality of conductors,
- compressing the subassembly, particularly so as to remove voids between the layer and the conductors of said plurality of conductors, and
- separating (particularly cutting) the subassembly into a plurality of disc-shaped electrical feedthroughs, such that each electrical feedthrough comprises a plurality of conductors electrically insulated from one another by the insulating material and extending from a front side to a back side of the respective electrical feedthrough, wherein the electrically insulating material encloses the respective conductor laterally in a hermetically sealed fashion.

Preferably, in an embodiment, winding of the layer is performed such that the subassembly and the respective final electrical feedthrough comprise a cylindrical shape, respectively. Particularly, compressing the subassembly is performed with an appropriate pressure that particularly may be chosen with regards to the properties of the layer of an electrically insulating material such as constituents (e.g., green sheet components, binder, solvent), moisture and the like. Appropriate pressures may lie in the range of 0.25 MPa to 0.75 MPa.

Further, according to an embodiment of the method according to the present invention, the electrically insulating material is one of or comprises one of:
- aluminium oxide (Al₂O₃),
- zirconium dioxide (ZrO₂),
- a glass,
- a glass composed of 45 wt% SiO₂, 24.5 wt% CaO, 24.5 wt% Na₂O, and 6.0 wt% P₂O₅ (e.g. Bioglass),
- a glass comprising lanthanum,
- a ceramics,
- a ceramics containing apatite and wollastonite as predominant crystalline phases, (e.g. an apatite-wollastonite (AW) glass-ceramics),
- silicon nitride (Si₃N₄),
- silicon carbide (SiC), and/or
- aluminium nitride (AlN).

Furthermore, according to an embodiment of the method, said layer is a ceramics tape, particularly an alumina green tape.

Furthermore, according to an embodiment of the method, the step of providing a plurality of conductors corresponds to arranging a plurality of conductors on an outer surface of a (preferably cylindrical) rod such that each conductor extends along a longitudinal axis of the rod, particularly parallel to one another. The conductors may be fixed to the rod in a suitable manner, e.g. by pressing them into the rod or pressing or drawing them through a capillary. Alternatively, the conductors may be made and thereby arragend by a photolithographic process including depositing a metal layer and etching away portions of the layer so as to form the individual conductors. According to a further alternative, the conductors may printed, e.g. onto the rod, and thereby arranged.

In an embodiment, this rod is formed out of or comprises the electrically insulating material (see e.g. above). Particularly, the rod can be formed out of a ceramics or alumina or another suitable electrically insulating material.

Furthermore, according to an embodiment of the method, the method comprises the further step of arranging a further plurality of conductors on an outer surface of said wound layer and winding a further layer of said electrically insulating material around said further plurality of conductors before said compressing, and compressing the further layer and said further plurality of conductors enclosed by the further layer, particularly so as to remove voids between the further layer and the conductors of said further plurality of conductors.

Also here, in an embodiment, said further layer is a ceramics tape, particularly an alumina green tape.

Particularly, in case a single layer of the electrically insulating material has been wound onto the rod, the method can comprise the further step according to which after compressing of the subassembly the subassembly is heated, particularly, so as to sinter the ceramics tape or alumina green tape when used as electrically insulating layer. Due to this heating, a hermetic sealing of the final feedthroughs can be achieved. Other electrically insulating materials that need heating (e.g. for sintering) may also be heated at this point. In some embodiments, the subassembly is heated to a temperature in the range of 600°C to 800°C, particularly in case of the ceramic tape comprises a LTCC (low-temperature-co-fired-ceramic).. In some embodiments, the subassembly is heated to a temperature of at least 1200°C, particularly in case of the ceramic tape comprises a HTCC (temperature-co-fired-ceramic). In some embodiments, the subassembly is heated to a temperature of approx. 1000°C, in case of the subassembly comprises alumina green tape.

The aforementioned heating may be conducted before or after said separating the subassembly into several electrical feedthroughs. Preferably, the heating is conducted before said separating.

Alternatively, as mentioned above, a further layer of an electrically insulating material may be wound onto the preceding layer. In this case, in an embodiment, the method may comprise the further step according to which after compressing of the further layer and said further plurality of conductors enclosed laterally by the further layer and before said separating, the entirety of layers and conductors can be heated for achieving an hermetic sealing of the final feedthroughs, particularly so as to sinter the ceramics tapes or alumina green tapes (or other materials that need heating/sintering, see above).

In other words, the compressed feedthrough rod can have additional pluralities of conductors and layers of electrically insulating material added to the circumference, to achieve a sufficient quantity of interconnects. Finally, the cylindrical assembly can be fired to e.g. sinter the alumina green tape (or alternative material stated herein) and cut into discs of sufficient thickness to create hermetic electrical feedthroughs.

Furthermore, according to an alternative embodiment of the method, the step of providing a plurality of conductors corresponds to arranging a plurality of conductors on a surface of a spread (e.g. flat) layer of an electrically insulating material. Particularly, the conductors are arranged such on this layer that they extend parallel to one another. The conductors can be pressed into the layer or fixed to the layer to simplify the winding process.

Here, according to an embodiment, winding said layer corresponds to winding the layer in a spiral fashion with the conductors arranged thereon such that the layer encloses said plurality of conductors laterally to form a subassembly comprising said (spirally wound) layer and said plurality of conductors therein. Particularly, the wound layer comprises several windings forming a spiral and enclosing said plurality of conductors laterally by way of said winding.

Summarizing, in the embodiment using a spirally wound layer, the method preferably comprises the steps of:
- arranging a plurality of conductors on a surface of a spread layer of the electrically insulating material,
- winding the layer in a spiral fashion with the conductors arranged thereon such that the layer encloses said plurality of conductors laterally to form a subassembly comprising said layer and said plurality of conductors.

According to an embodiment, after compressing of the (spirally wound) subassembly (particularly to ensure that no voids are present between the conductors and the wound layer), the subassembly is heated for achieving a hermetic sealing of the final electrical feedthroughs. Particularly, said heating causes sintering of the ceramics tape or alumina green tape (or of an alternative electrically insulating material stated herein).

Particularly, according to an embodiment, the conductors of the respective electrical feedthrough are formed out of or comprise one of the following materials: palladium (Pd), platinum (Pt) platinum/iridium, niobium, gold, nickel or copper.

Further, according to an embodiment of the method according to the present invention, each conductor of an electrical feedthrough comprises an exposed face side on a front side of the feedthrough and an exposed face side on a back side of the feedthrough. The face sides on the front side are preferably flush with the front side and the faces sides on the back side are preferably flush with the back side of the electrical feedthrough (e.g. by way of separating the individual electrical feedthrough from the subassembly).

According to an embodiment, a tab of a solderable material is laser welded to the face side of each conductor on the back side of an electrical feedthrough separated from the subassembly.

According to a further embodiment, an electronic module of an implantable medical device e.g. a cardiac pacemaker), particularly a printed circuit board assembly (PCBA), is soldered to the tabs. Particularly, the respective tab can be formed out of copper (Cu), wherein the respective tab can comprise a surface plating such as ENIG (for Electroless Nickel Immersion Gold). Such an ENIG plating consists of an electroless nickel plating covered with a layer of immersion gold.

According to a further embodiment of the method according to the present invention, the electrical feedthrough is brazed to a flange, particularly made of or comprising titanium or a titanium alloy, and the flange is laser welded to a housing, particularly a titanium housing, of the implantable medical device. Brazing as described above is preferably performed with gold as a solder.

According to a further embodiment of the method according to the present invention, a header wiring element of a header of the implantable medical device is laser welded to the face side of each conductor on the front side of the feedthrough.

Particularly, the header is configured to connect at least one component (e.g. an electrode lead) to the implantable medical device (e.g. cardiac pacemaker), so as to connect the component to an electronic module of the implantable medical device that is enclosed by the housing.

A further aspect of the present invention relates to an electrical feedthrough produced with the method according to the present invention.

Furthermore, yet another aspect of the present invention relates to an electrical feedthrough for an implantable medical device, wherein the electrical feedthrough comprises a plurality of conductors enclosed by an electrically insulating body, wherein each conductor comprises an exposed face side on a front side of the feedthrough and an exposed face side on a back side of the feedthrough, wherein the body comprises a wound and sintered layer of an electrically insulating material laterally enclosing the conductors.

Particularly, the insulating material directly contacts each of the plurality of conductors, i.e. without a solder or the like between the conductor and the insulating material.

According to a further aspect of the present invention, an implantable medical device is disclosed, particularly an implantable cardiac pacemaker, comprising an electrical feedthrough according to the present invention (e.g. an electrical feedthrough comprising the afore-mentioned features or an electrical feedthrough produced with the method according to the present invention).

According to an embodiment of the implantable medical device, a tab is connected, particularly laser welded, to the face side of each conductor on the back side of the feedthrough. Particularly, the respective tab can be formed out of copper (Cu), wherein the respective tab can comprise a surface plating such as ENIG (see also above).

According to a further embodiment of the implantable medical device, the implantable medical device comprises an electronic module (particularly a printed circuit board assembly (PCBA)) that is connected, particularly soldered, to the tabs.

According to a further embodiment of the implantable medical device, the implantable medical device comprises a housing for accommodating the electronic module. Particularly, the housing comprises titanium or a titanium alloy or is formed out of titanium or a titanium alloy. Alternatively, the housing may comprise a biocompatible plastic such as LCP, silicone or epoxy or may be formed out of the biocompatible plastic.

According to a further embodiment of the implantable medical device, the electrical feedthrough is connected to the housing via a flange. Particularly, the flange is formed out of titanium or a titanium alloy or comprises titanium or a titanium alloy.

Particularly, according to an embodiment of the implantable medical device, the electrical feedthrough is brazed to the flange, particularly with gold as a solder. Furthermore, particularly, the flange is laser welded to the housing.

According to a further embodiment of the implantable medical device, the implantable medical device comprises a header configured to provide an electrical connection between a component of the implantable medical device (such as an electrode lead, e.g. in case the implantable medical device is an implantable cardiac pacemaker) and the electronic module or PCBA enclosed by the housing of the implantable medical device.

Further, according to an embodiment of the implantable medical device, the header comprises header wiring elements for electrically connecting the header to the conductors of the electrical feedthrough, wherein each header wiring element is laser welded to the face side of a conductor of the electrical feedthrough on the front side of the electrical feedthrough.

In the following, embodiments of the present invention as well as further features and advantages of the present invention shall be described with reference to the Figures, wherein
- Fig. 1: shows an embodiment of a method for producing an electrical feedthrough according to the present invention;
- Figs. 2A to 2B: show a front side (Fig. 2A) and a back side (Fig. 2B) of an electrical feedthrough manufactured according to the method illustrated in Fig. 1;
- Figs. 3 to 5: show a further embodiment of a method for producing an electrical feedthrough according to the present invention; and
- Fig. 6: shows a cross sectional view of a single conductor of an embodiment of an electrical feedthrough according to the present invention.

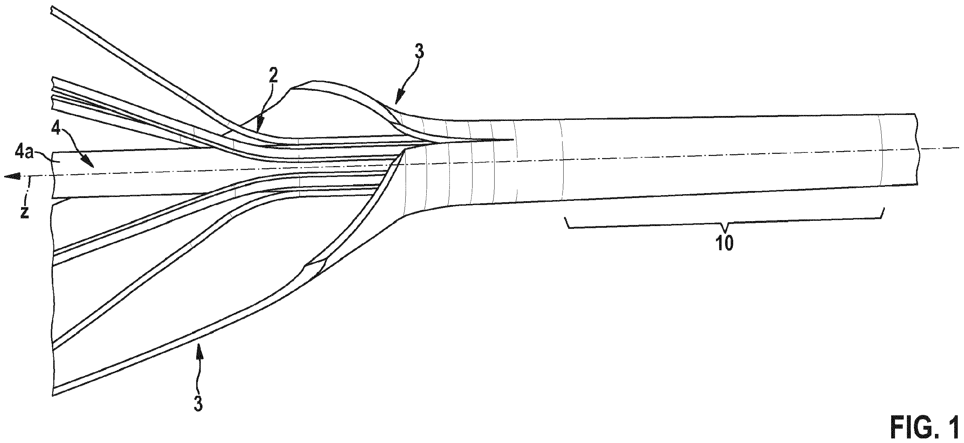

Fig. 1 illustrates an embodiment of a method for manufacturing an electrical feedthrough 1 for an implantable medical device. As shown in Fig. 1, a plurality of conductors (e.g. in form of palladium or platinum wires) 2 is provided, particularly by arranging the individual conductor 2 on an outer surface 4a of a rod 4 such that the conductors extend along a longitudinal axis z of the rod 4 as well as parallel to one another. Then a layer 3 comprising an electrically insulating material, such as alumina, is placed on the conductors 2 so that the latter are covered along the circumference of the rod 4. The layer 3 can be an alumina greed tape. The rod 4 can also be formed out of alumina. Other electrically insulating materials can also be used for the layer 3 and rod 4.

Due to winding the layer 3 on the conductors 2, the latter are laterally enclosed by the layer 3. In order to get rid of voids between the conductors 2 and the layer 3 / rod 4, the subassembly 10 consisting of the conductors 2, wound layer 3 and rod 4 is compressed (see right hand side of Fig. 1).

The compressed rod 4, conductors 2, and layer 3 can then have additional layers of conductors (e.g. palladium or platinum wires) 2 and alumina green sheet 3 added to the circumference to achieve the sufficient quantities of interconnects. The cylindrical assembly is finally fired to sinter the alumina green tape and cut into disks of sufficient thickness to create corresponding hermetic electrical feedthroughs 1.

Thus, the method allows manufacturing hermetic electrical feedthroughs 1 in a semi-continuous process flow using e.g. ceramic green (e.g. alumina) tape and conductors (e.g. metal traces or wire comprising palladium or platinum) in/on the green tape.

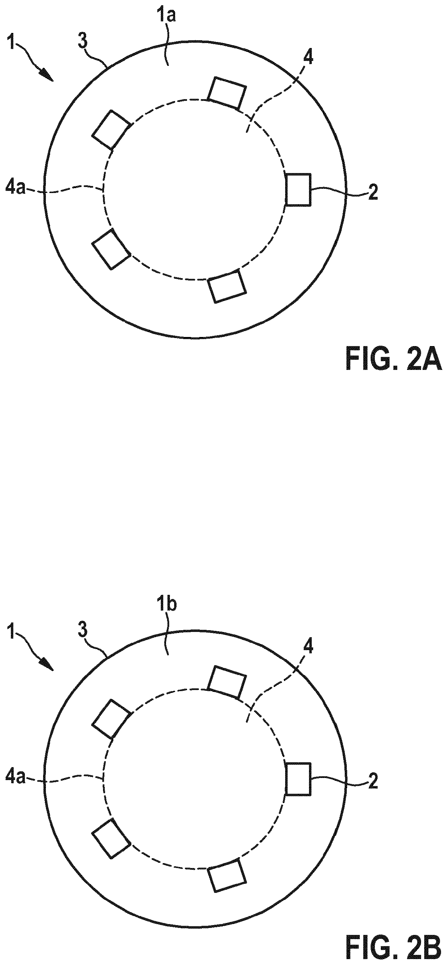

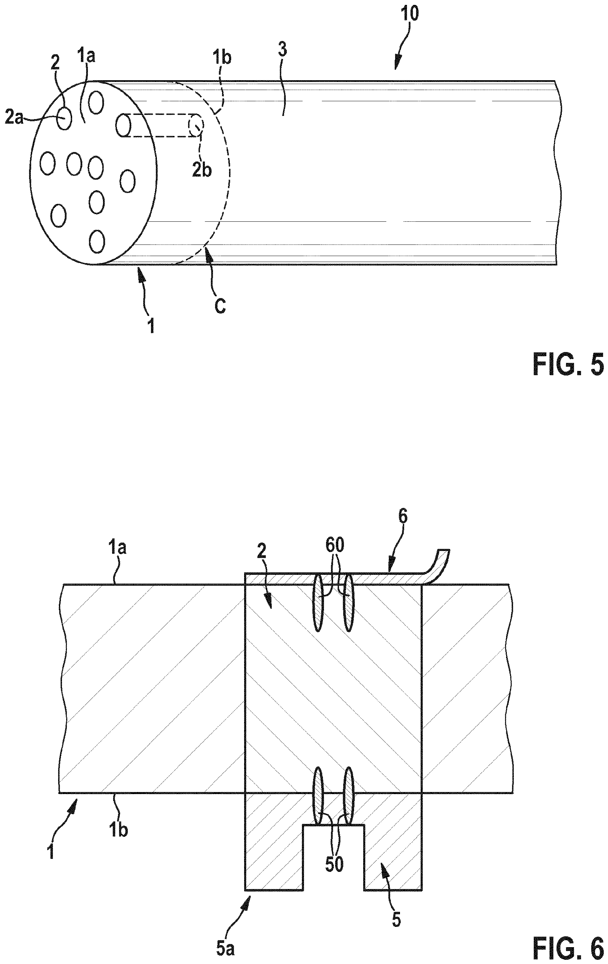

The respective final electrical feedthrough 1 now comprises according to Figs. 2A and 2B a front side 1a having exposed face sides 2a of the conductors 2 (cf. Fig. 2A) as well as a back side 1b having exposed face sides 2b of the conductors 2. The face sides 2a, 2b of the conductors 2 can then be electrically contacted as will be described in more detail below in conjunction with Fig. 6.

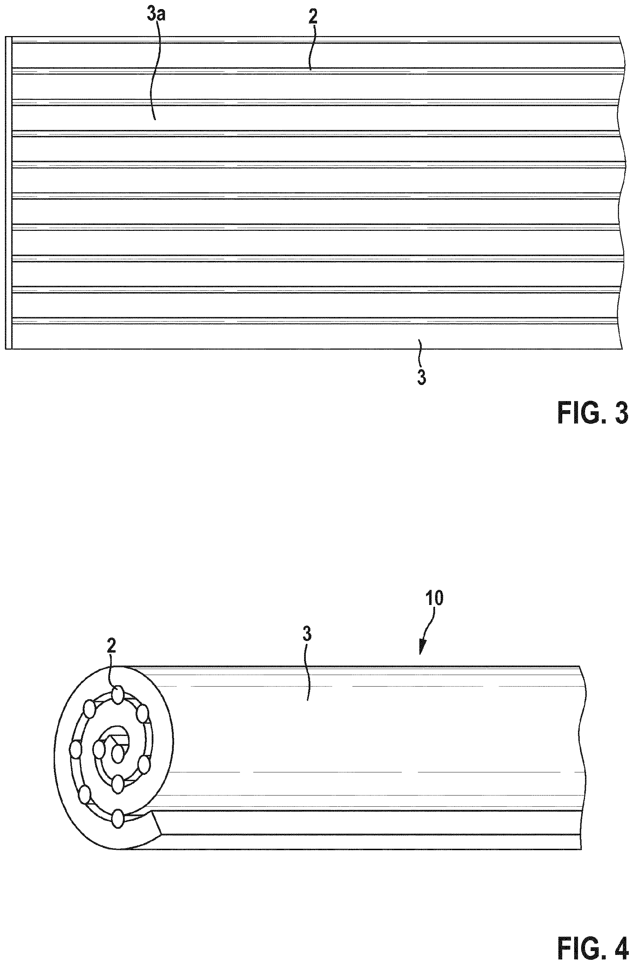

Figs. 3 to 5 show a further embodiment of the method according to the present invention. Here, the electrical feedthroughs 1 are created by spiral rolling of a layer 3 of an electrically insulating material (e.g. an alumina green tape). As shown in Fig. 3 the conductors 2 (e.g. palladium or platinum wires) are arranged on a surface 3a of the spread layer 3. Particularly, the conductors 2 (Pt, Pd, etc.) are pressed into and distributed along the length of the layer 3, wherein a spacing between the conductors 2 can be constant (as shown in Fig, 3), but may also be variable depending e.g. on design optimization.

Once the length of the layer 3 has been rolled out and the conductors 2 spaced and pressed into it, the layer 3 (e.g. ceramic or alumina green tape) and conductors 2 thereon can be cut to a desired size if necessary. Thereafter, the layer 3 is rolled to form a spiral thereby enclosing the conductors 2 as shown in Fig. 4.

This subassembly 10 is then compressed to remove voids and heated to sinter the electrically insulating material to yield a hermetic structure 10 shown in Fig. 5. This compressed and heated subassembly 10 can then be separated into individual electrical feedthroughs 1 (e.g. by cutting along the dashed line C in Fig. 5) as already mentioned above. These feedthroughs 1 then comprise a front side 1a and a back side 1b having exposed face sides 2a, 2b (cf. also Fig. 2A and 2B) of the conductors 2 that can be electrically contacted.

While connecting directly to the face sides 2a, 2b of the conductors 2 is possible, due to the formation of brittle intermetallics of Pd and Sn it is not preferred to form a direct solder connection to the exposed surfaces of the hermetic feedthrough's 1 conductors 2. To enable connection of the feedthrough 1 to an electronic module or printed circuit boards assembly (PCBA) in the medical device, one approach shown in Fig. 6 is to laser weld a tab 5 of a solderable material (e.g. such as Cu with an ENIG surface finish) to the face side 2b of the respective conductor 2 on the back side 1b of the feedthrough 1, which tab 5 is particularly of the same size as the exposed face side 2b. Fig. 6 also indicates the corresponding weld seam 50.

The respective tab 5 then forms the solder joint with the electronic module/PCBA. Particularly, the process of attaching the respective tab 5 to the cylindrical disc feedthrough 1 can be automated with use of alignment fixtures and laser welding on an x-y stage with multiple feedthroughs 1 on the fixture.

The feedthrough disks 1 can then be processed to braze the feedthrough 1 to a titanium or titanium alloy flange and allow for laser welding onto a titanium or titanium alloy housing of a the implantable medical device.

The connection with an external header wiring element 6 could be formed by laser welding the wiring element 6 to the corresponding face side 2a of a conductor 2 on the front side 1a of the feedthrough 1, as shown in Fig. 6. Also shown is the weld seam 60 between the wiring element 6 and the conductor 2.

The present invention advantageously allows use of continuous components for manufacture of electrical feedthroughs by e.g. suitably rolling of e.g. ceramic green sheet 3 and metal traces 2 to yield a cylindrical shaped electrical feedthrough 1 with a simple manufacturing method.

The electrical feedthroughs produced in this fashion can comprise a high interconnect density. Furthermore, a highly automatable semi-continuous process flow with reducing manufacturing complexity can be used. Furthermore, processing and costs of other components, such as the titanium flange, can be reduced due to the achievable cylindrical shape of the feedthrough.

## Claims

1. A method for producing an electrical feedthrough (1) for a medical device, comprising the steps of:
- providing a plurality of conductors (2),
- winding a layer (3) comprising an electrically insulating material, such that the layer (3) encloses said plurality of conductors (2) to form a subassembly (10) comprising said layer (3) and said plurality of conductors (2),
- compressing of the subassembly (10), and
- separating the subassembly (10) into a plurality of disc-shaped electrical feedthroughs (1).

2. The method according to claim 1, wherein the electrically insulating material is one of or comprises one of:
- aluminium oxide,
- zirconium dioxide,
- a glass,
- a glass composed of 45 wt% SiO₂, 24.5 wt% CaO, 24.5 wt% Na₂O, and 6.0 wt% P₂O₅,
- a glass comprising lanthanum,
- a ceramics,
- a ceramics containing apatite and wollastonite as predominant crystalline phases,
- silicon nitride,
- silicon carbide,
- aluminium nitride.

3. The method according to one of the preceding claims, wherein said layer (3) is a ceramics tape, particularly an alumina green tape.

4. The method according to one of the preceding claims, wherein the step of providing a plurality of conductors (2) corresponds to arranging a plurality of conductors (2) on an outer surface (4a) of a rod (4) such that each conductor (2) extends along a longitudinal axis (z) of the rod (4).

5. The method according to claim 4, wherein the rod (4) is formed out of or comprises the electrically insulating material.

6. The method according to one of the preceding claims, wherein the method comprises the further step of arranging a further plurality of conductors on an outer surface of said layer and winding a further layer comprising said electrically insulating material around said further plurality of conductors before said compressing, and compressing the further layer and said further plurality of conductors enclosed by the further layer.

7. The method according to claim 6, wherein said further layer is a ceramics tape, particularly an alumina green tape.

8. The method according to one of the claims 1 to 5, wherein after compressing of the subassembly (10), and particularly before said separating, the subassembly (10) is heated.

9. The method according to claim 6 or 7, wherein after compressing of the further layer and said further plurality of conductors enclosed by the further layer and before said separating, the entirety of layers and conductors is heated.

10. The method according to one of the claims 1 to 3, wherein the step of providing a plurality of conductors (2) corresponds to arranging a plurality of conductors (2) on a surface (3a) of a spread layer (3) comprising an electrically insulating material.

11. The method according to claim 10, wherein the step of winding said layer (3) corresponds to winding the layer (3) in a spiral fashion with the conductors (2) arranged thereon such that the layer (3) encloses said plurality of conductors (2) to form a subassembly (10) comprising said layer (3) and said plurality of conductors (2).

12. The method according to claim 10 or 11, wherein after compressing of the subassembly (10) the subassembly (10) is heated.

13. The method according to one of the preceding claims, wherein the conductors (2) are formed out of or comprise one of the following materials: palladium, platinum, platinum/iridium, niobium, gold, nickel or copper .

14. An electrical feedthrough (1) produced with the method according to one of the preceding claims.

15. An electrical feedthrough (1) for an implantable medical device, comprising a plurality of conductors (2) enclosed by an electrically insulating body (3), wherein each conductor (2) comprises an exposed face side (2a) on a front side (1a) of the electrical feedthrough (1) and an exposed face side (2b) on a back side (1b) of the electrical feedthrough (1), wherein the body (3) comprises a wound and sintered layer of an electrically insulating material enclosing the conductors (2), wherein particularly the electrically insulating body (3) directly contacts each conductor (2).
